# EUROPEAN PATENT SPECIFICATION

(11) **EP 0 773 777 B1**
(45) Date of publication and mention of the grant of the patent: **24.03.1999**
(21) Application number: 94903149.6
(22) Date of filing: 17.12.1993
(51) Int. Cl.: A61J 15/00, A47G 21/18

(54) **A DRINKING SYSTEM AND MOUTHPIECE FOR USE THEREIN**
TRINKSYSTEM UND MUNDSTÜCK ZUR VERWENDUNG DAMIT
SYSTEME A BOIRE ET EMBOUT ASSOCIE

(30) Priority: 18.12.1992 NL 9202202
(43) Date of publication of application: 21.05.1997
(73) Proprietor: VAN EENENNAAM, Johannes Willem Salomon, NL-7316 BT Apeldoorn (NL); RAKHORST, Gerhard, NL-9722 SN Groningen (NL); VERKERKE, Gijsbertus Jacob, NL-9753 EP Haren (NL)
(72) Inventor: RAKHORST, Gerhard, NL-9722 SN Groningen (NL); VERKERKE, Gijsbertus, Jacob, NL-9753 EP Haren (NL)
(74) Representative: Tan, Adriaan Hoan-Kim
(86) International application number: NL9300271
(87) International publication number: WO9414400

(56) References cited:
- FR-A- 2 258 164
- FR-A- 2 387 030
- GB-A- 2 067 416
- US-A- 3 165 241
- US-A- 4 196 747
- US-A- 4 969 564

## Description

The invention relates to a drinking system comprising a channel for passing liquid from a container to the user's mouth and a check valve arranged in the channel for passing liquid exclusively in a specific direction of flow.

Such a drinking system is disclosed in U.S. patent 4,196,747. Another example of such a drinking system is disclosed in U.S. patent 5,031,831.

In the practice of these drinking systems, the liquid is passed to the user's mouth by suction. Such drinking systems for bringing liquids to one's mouth can be used not only in a festive or recreational environment, but also, with advantage, in medicine and sports, where frequently situations occur where it is impossible or objectionable to bring the container with potable liquid or the like to the user's mouth or the position of the user or the circumstances make it difficult to drink directly from a container. An important application in the field of health care is to afford a bedridden patient an opportunity to drink without having to sit up for the purpose. An important application in the field of sports is to afford a cyclist on a cycle an opportunity to ingest moisture with minimum loss of time, for instance during a time trial in cycling, or during the cycling phase of a triathlon, where getting hold of the water bottle and raising himself to drink from it may entail so much delay that the cyclist achieves a lower placing. During motor and motorcycle races, too, the race car driver or motor cyclist is not generally in a position to drink from a container.

The above-described systems being equipped with a check valve prevents the situation where each time drinking is resumed, first a considerable amount of air has to be sucked from the channel before the liquid reaches the mouth. The use of a check valve further prevents contamination and fouling of the channel, the container and the liquid supply as a result of liquid flowing back from the user's mouth, which entails the danger of infection.

These known drinking systems, however, present a problem in that liquid regularly flows from the end of the channel to be held in the mouth at undesired moments, for instance when the conduit is put down in flat orientation, carbonated liquid is present in the channel or the conduit through which the channel extends is bent or compressed.

U.S. Patents 2,969,064, 3,165,241, 4,782,975 and 5,085,349 and French patent application 2,387,030 disclose drinking systems comprising a container and a channel for passing potable liquid from the container to the mouth and wherein a user-operable valve is located near the end of the channel to be held in the mouth. Two further pertinent examples of drinking system, in the form of baby-bottles, are to be found described in GB-A-2 067 416 and in US-A-4 969 564. These drinking systems present a problem in that the coordination of the operation of the valve on the one hand and the ingestion and swallowing of the liquid on the other complicates the operation. If the container is located at a higher level than the end of the channel to be held in the mouth or if an overpressure prevails in the container, the chances of liquid spill or choking are relatively substantial with these drinking systems, choking in particular being most undesirable in the case of weakened patients or a great physical effort. If the container is located at a lower level than the end of the channel to be held in the mouth or if an underpressure prevails in the container, two categories of problems occur. First, upon suction no liquid is procured as long as the valve is not opened. Secondly, if the valve is opened but no suction or insufficiently hard suction is applied (for instance during swallowing), liquid will flow back through the channel into the container, so that contamination and fouling of the channel, the container and the liquid arises and, upon renewed suction, air has to be sucked from the channel before liquid can be sucked up.

The object of the invention is to provide a drinking system in which the above-mentioned problems do not occur.

This object is achieved by means of a drinking system of the type described in the preamble, having the features specified in the characterizing portion of claim 1.

Because the valve is arranged near the end of the channel to be held in the mouth, it is possible, upon a decrease of the volume of the channel, caused, for instance, by bending or compression of a conduit through which the channel extends, or upon an increase of the volume of the liquid in the channel, for instance as a result of carbon dioxide being liberated from the liquid, for the liquid to move in the direction of the container and to flow from the channel, so that it will not flow from the end of the channel to be held in the mouth. In spite of the fact that liquid can freely pass the check valve in the direction of the end of the channel to be held in the mouth, it will always tend to flow to the container because the container is arranged at a lower level than the end of the channel to be held in the mouth and therefore will not flow from the end of the channel to be held in the mouth in the case of the above-mentioned variations in volume.

The check valve prevents the reflux of liquid which has passed beyond the check valve, so that contamination and fouling of the channel can yet be prevented. Moreover, if no drinking is taking place, the check valve forms a closure of the channel near the downstream end thereof, so that a barrier against contamination of the channel, the container and the liquid is formed, in particular through the prevention of diffusion of particles past the valve in the longitudinal direction of the channel.

Further, in use the check valve, in combination with the liquid column depending therefrom, automatically forms a closure of the channel near the downstream end thereof. This prevents the situation where, upon the channel being put down in flat orientation, the meniscus of the liquid at the downstream end of the channel yields and the liquid can flow uncontrollably from a channel section adjacent the end of the channel to be held in the mouth.

When drinking is interrupted, the liquid in the channel is automatically retained with the check valve. As a result, when drinking is interrupted, the liquid does not flow from the channel and drinking can always be resumed without requiring that the channel be first sucked full of liquid again; to permit the liquid present in the container to be drunk the channel need only be filled with liquid once and the channel does not become empty until the liquid in the container has been used up and the last liquid reaches the user's mouth.

Because the use of the drinking system according to the invention permits the container to be arranged lower than the downstream end of the channel, no special device for keeping the container above the user's head is necessary. Liquid which has been introduced into the channel cannot flow back to the container because it remains suspended behind the check valve in the channel.

The drinking system according to the invention can be used to drink from an open mug or the like. However, a substantially airtight container, which is for instance used to avoid loss of liquid through shaking or turnover and deterioration of the liquid, should be adapted for use in a drinking system according to the invention. In the known drinking system, air could flow into the container through the channel. In the drinking system according to the invention, this is not possible because the liquid remains standing in the channel.

Accordingly, it is advantageous to equip the drinking system according to the invention with an adapted drinking cup which comprises not only a substantially airtight and form-retaining tank and a passage for the channel, but also an aeration opening. If the container is not form-retaining and is designed as a bag, for instance, an aeration opening is not necessary because the container has a variable volume which is capable of following the decrease of the amount of liquid present in the container.

An additional advantage of his drinking cup is that it enables continuous drinking whilst the known airtight container requires that drinking be interrupted repeatedly so as to allow access of air to the container.

The invention may be further embodied in a mouthpiece adapted for use in a drinking system according to the invention, which mouthpiece comprises a passage extending from a connection for connecting a conduit and a check valve arranged in the passage, the check valve being adapted for passing liquid exclusively in a direction away from the connection. With this mouthpiece according to the invention, a standard conduit can be simply converted to a drinking system according to the invention by connecting the mouthpiece to the conduit. The mouthpiece can be manufactured in a large variety of appearances and may for instance be incorporated into a comic character to render its use more appealing to children.

Hereinafter the invention will be explained in detail on the basis of some exemplary embodiments, with reference to the drawings. In the drawings:
Figs. 1 and 2 are cut-off sectional elevations of a first and a second exemplary embodiment of a drinking system according to the invention;
Figs. 3-5 are cut-off side elevations of a third, a fourth and a fifth exemplary embodiment of a drinking system according to the invention; and
Figs. 6-9 are cross-sectional side elevations of different containers for use in a drinking system according to the invention.

In the drawings corresponding parts of the different exemplary embodiments have been designated by identical reference numerals.

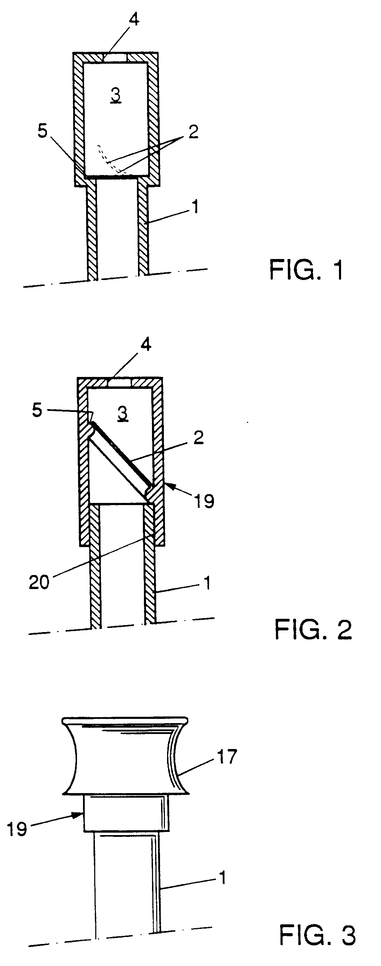

The drinking system according to the invention shown in Fig. 1 comprises a channel 1 for passing liquid from a container to the user's mouth. The channel 1 comprises a check valve 2 accommodated in a chamber 3. The closed position of the check valve 2 is indicated by solid lines and the open position by dotted lines. Fig. 1 only depicts the section of the channel adjacent the downstream end to be held in the mouth.

In use the container should be located at a lower level than the end of the channel 1 to be held in the mouth. Prior to drinking the other end should be inserted into the container filled with potable liquid and the liquid is to be passed to the end of the channel 1 to be held in the mouth. In most cases this will be realised most readily by simply sucking up the potable liquid. In the case where this is objectionable, it is also possible to allow the liquid to flow into the channel 1 by keeping the end of the channel opposite the oral end sufficiently high and allowing the liquid to flow into the channel or by producing an overpressure in the container accommodating the liquid. While the liquid is being sucked up, the check valve 2 is opened, but as soon as suction is interrupted, the check valve 2 closes, so that the reflux of liquid into the channel 1 is prevented and a column of liquid remains suspended in the channel behind the check valve.

It is therefore not necessary each time drinking has been interrupted to suck the air from the channel 1 again before liquid reaches the mouth. Moreover, it is not necessary that during swallowing the underpressure be maintained in the mouth or the channel be closed so as to prevent reflux of liquid into the channel.

In practice, the channel 1 generally has a length of 0.25-1.25 m. Because the container is arranged lower than the downstream end of the channel 1, the liquid in the channel 1 will tend to flow back to the container. This is prevented by the check valve in the channel.

After-drip from the channel is prevented by virtue of the channel, at the downstream end thereof, being provided with a constriction 4, which is so narrow that a stable meniscus is formed therein.

The check valve 2 is located near the end of the channel to be held in the mouth. As a consequence, the liquid can escape to the container in the case of a decrease of the volume of the channel 1 or in the case of an increase of the volume of the liquid contained therein. Such changes in volume may for instance occur as a result of the channel being squeezed, changes in temperature or the liberation of gasses from the potable liquid (in particular when carbonated). Strictly speaking, no change in the volume of the liquid is then involved but a change in the volume of a liquid-gas mixture, but for brevity this mixture is designated as liquid.

Because the check valve 2 is designed as a flexible membrane, it reacts very quickly irrespective of the position of the part of the channel 1 where the check valve 2 is located. Such a check valve can moreover be manufactured at low cost and provides a reliable seal by virtue of its flexibility.

The drinking system further comprises a shoulder forming a seat 5 against which abuts the valve 2 in closed condition. Thus, the reliability of the seal is further increased and a considerable clearance between the valve 2 and the wall of the chamber 3 is allowable. Accordingly, the valve 2 need not be manufactured with great accuracy and in spite of that will not abut against the wall of the chamber 3 when being opened and closed.

In the drinking system depicted in Fig. 2, as in the drinking system depicted in Fig. 1, the membrane 2 is connected on one side with the wall of a passage in which it is arranged. However, in closed condition the membrane 2 extends from that side obliquely towards the opposite side of the passage and in downstream direction towards the end of the channel 1 to be held in the mouth. As a result, during opening and closing the membrane 2 need bend to a lesser extent than the membrane depicted in Fig. 1.

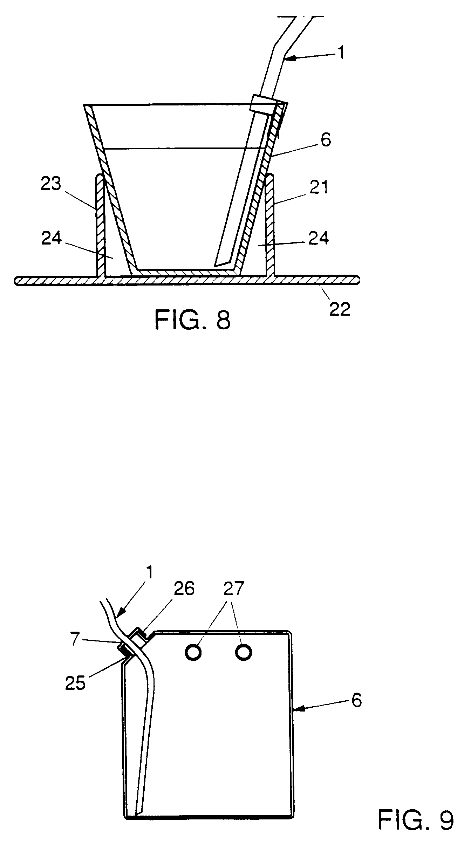

The drinking system according to the invention can be used in combination with a conventional container, such as the cup-shaped container 6 shown in Fig. 8. To prevent the cup tipping over, it can be placed in a support 21. This provides the further advantage that channel 1 can be attached to the edge of the container 6 without giving rise to an undue risk of the container 6 tipping over as a result of forces exerted by way of the channel 1. The support 21 comprises a bottom portion 22 and a shell-shaped upright portion 23, and flanges 24 which extend radially inwards from the shell-shaped upright portion 23 and whose configuration is adapted to the shape of the container 6. The support 21 can be simply manufactured by injection-moulding and a common disposable cup, for instance, can be used as a container.

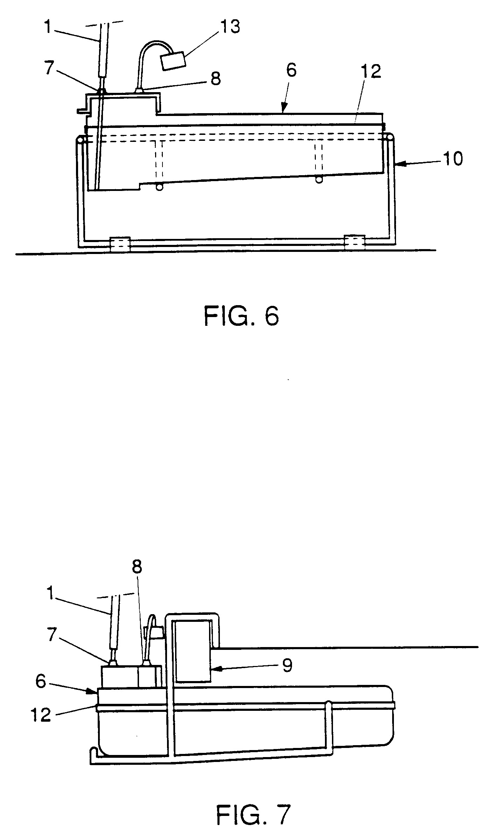

In many cases, however, it is desired for the container to be substantially airtight, for instance to prevent decay or cooling of the potable liquid or to avoid loss of liquid upon abrupt movements. Figs. 6 and 7 depict substantially form-retaining and airtightly closed containers 6 which are adapted for use in a drinking system according to the invention, which does not allow of aeration through the channel 1. These containers 6 comprise a passage 7 for the channel 1 and an aeration opening 8 for aerating the container 6. As a result, the contents of the container can be drunk up without interruption without an undue underpressure being produced in the container.

The containers 6 according to Figs. 6 and 7 have a relatively large length and width, so that they do not easily tip over and do not project far when attached to a bed 9, for instance (see Fig. 7).

Fig. 9 depicts a container 6 which is not form-retaining but is designed as a bag. This container 6 further comprises a filling hole 25 provided with a cap 26 and suspension eyes 27. The cap 26 comprises a passage for the channel 1. This container 6 does not comprise an aeration opening. An aeration opening is not necessary in this container because the bag 6 is so flexible and formed in such a manner that it properly follows the volume that decreases as the liquid is being withdrawn.

The container shown in Fig. 6 is placed in a rack 10, which may for instance be arranged on the floor. The container shown in Fig. 7 is placed in a rack 11 which is adapted to be suspended from the edge of the bed 9. The containers 6 are each divisible along a joint 12, so that they can be cleaned easily. If it is desired chat the surface of the liquid that is in contact with the ambient air be kept small, it is of course also possible to use a container of slight width and length but of relatively great height.

The aeration passage 8 of the container 6 depicted in Fig. 6 communicates with a filter 13. Air being drawn in is treated by the filter. This prevents fouling and contamination of the potable liquid.

The aeration opening can also be used for producing an overpressure in the container and the channel in order to fill the channel with liquid prior to drinking.

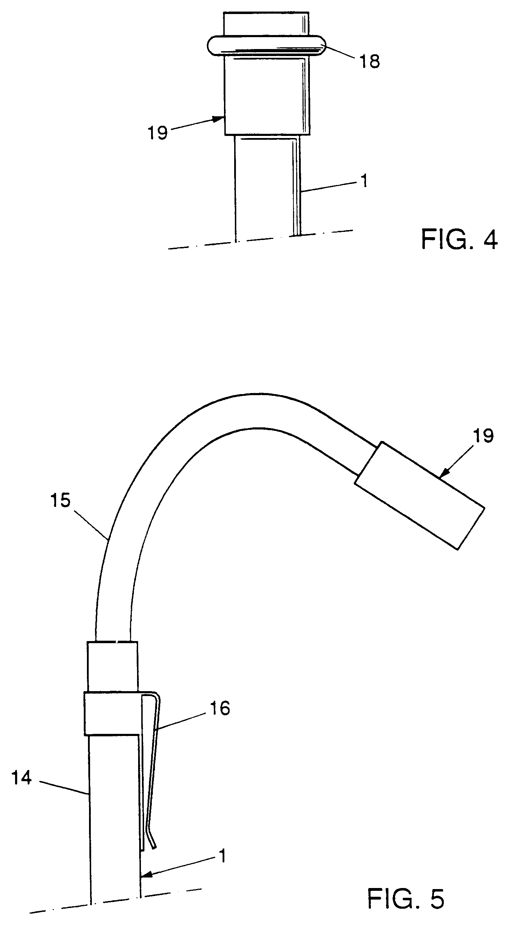

The channel 1 of the drinking system depicted in Fig. 5 comprises a form-retaining conduit portion 14 and a flexible conduit portion 15. Arranged adjacent the end of the fixed portion 14 on the side of the downstream end of the channel 1 is an attachment member 16 for attaching the channel 1 to the user or near the user. Thus the flexible portion can at all times be kept in the vicinity of the mouth of the user, who can easily bend it towards his mouth to drink. Of course the attachment member 16 can also be used in combination with an entirely flexible or entirely form-retaining channel.

The attachment member shown is designed in accordance with a clip of a ball pen. Depending on the application, other designs may be advantageous too. The attachment member may for instance be provided with a strap to be suspended from the neck or a bracket to be attached to a helmet.

The drinking system depicted in Fig. 3 is provided with a recess 17 on the outside of the channel 1 in the area of the end of the channel 1 to be held in the mouth. By virtue of this recess the channel 1 is easily retained in the user's mouth and for that purpose he only needs to keep his lips pressed towards each other with a relatively slight force.

The drinking system depicted in Fig. 4, too, is adapted to be readily retained in the mouth. In this drinking system, this has been realised through an upright ridge 18, which can be retained behind the teeth, arranged in the area of the end of the channel 1 to be held in the mouth.

The drinking systems shown in Figs. 2-5 comprise a conduit having connected thereto a mouthpiece 19 incorporating the check valve 2. The mouthpiece 19 is provided with a passage 3,4 where the check valve is arranged and a connection for connecting a conduit. In the mouthpieces 19 as shown, the connection for connecting a conduit is designed as a hole 20 (see Fig. 2) in which the conduit can be inserted. This can of course be designed differently as well, for instance as a tubular part which can be inserted into the conduit.

The mouthpieces 19 can each be simply coupled to a conduit of the desired type and of the desired length and after use be discarded or be cleaned separately and optionally sterilised. Moreover, by means of the mouthpieces it is possible to convert existing drinking systems with a conduit to drinking systems according to the invention.

The drinking system according to the invention can be advantageously used for administering potable liquids, but also more viscous substances such as yoghurt, custard and liquid food. The higher the viscosity of the liquid to be drunk, the greater the diameter of the channel should be, so as to limit the flow resistance in the channel.

## Claims

1. A drinking system comprising a channel (1) for passing liquid from a container (6) to a user's mouth and a check valve (2) arranged in the channel (1) for allowing liquid to pass exclusively in a specific direction of flow, said check valve (2) being arranged in a chamber near a downstream end of the channel (1) viewed in the direction of flow, said chamber having an inlet opening and an outlet opening and being bounded by walls, **characterized in that** the check valve (2) comprises a flexible membrane, and the chamber (3) is located directly adjacent the downstream end of the channel (1), such that the outlet opening forms the downstream end of the channel (1), an outside portion of the channel (1) directly adjacent its downstream end being formed by the walls bounding the chamber (3).

2. A drinking system according to claim 1, Comprising a shoulder with a seat (5) against which abuts the check valve (2) in closed condition.

3. A drinking system according to claim 2, wherein the membrane (2) is connected on one side with the wall of the chamber (3) in which it is arranged and in closed condition extends from that side obliquely towards the opposite side of the passage and the downstream end of the channel (1).

4. A drinking system according to any one of the preceding claims, comprising an airtightly closed container (6) with a passage for the channel (1) and an aeration passage (8).

5. A drinking system according to claim 4, wherein a filter (13) is arranged in the aeration passage (8).

6. A drinking system according to any one of the preceding claims, comprising a circumferential recess (17) on the outside of the wall of the chamber (3) and near the downstream end of the channel (1).

7. A drinking system according to any one of the preceding claims, comprising an outwardly projecting upright ridge (18) on the outside of the wall of the chamber (3) and near the downstream end of the channel (1).

8. A drinking system according to any one of the preceding claims, wherein the channel extends through a conduit and a mouthpiece connected to the conduit, the mouthpiece containing said chamber (3) in which the check valve (2) is arranged.

9. A mouthpiece adapted for use in a drinking system according to claim 8, comprising a passage (3, 4) extending from a connection for connecting a conduit to a downstream end and a check valve (2) for allowing liquid to pass exclusively in a specific direction of flow, said check valve (2) comprising a flexible membrane, and the chamber (3) being located directly adjacent the downstream end of the channel (1), such that the outlet opening forms the downstream end of the channel (1), an outside portion of the channel (1) directly adjacent its downstream end being formed by the walls bounding the chamber (3).

## Patentansprüche

1. Trinksystem mit einem Kanal (1) zum Leiten von Flüssigkeit aus einem Behälter (6) zum Mund eines Benutzers und einem in dem Kanal (1) angeordneten Rückschlagventil (2), das ermöglicht, daß die Flüssigkeit ausschließlich in eine bestimmte Fließrichtung fließt, wobei das Rückschlagventil (2) in einer Kammer nahe einem in Fließrichtung gesehen stromabwärtigen Ende des Kanals (1) angeordnet ist, wobei die Kammer eine Einlaßöffnung und eine Auslaßöffnung aufweist und durch Wände begrenzt ist, dadurch gekennzeichnet, daß das Rückschlagventil (2) eine flexible Membran aufweist, und die Kammer (3) dem stromabwärtigen Ende des Kanals (1) unmittelbar benachbart derart angeordnet ist, daß die Auslaßöffnung das stromabwärtige Ende des Kanals (1) bildet, wobei ein äußerer Bereich des Kanals (1) unmittelbar nahe dem stromabwärtigen Ende durch die die Kammer (3) begrenzenden Wände gebildet ist.

2. Trinksystem nach Anspruch 1, mit einer Schulter, die mit einem Sitz (5) versehen ist, an dem das Rückschlagventil (2) im Schließzustand anliegt.

3. Trinksystem nach Anspruch 2, bei dem die Membran (2) auf einer Seite mit der Wand der Kammer (3) verbunden ist, in der sie angeordnet ist, und daß sie sich im Schließzustand von dieser Seite aus schräg in Richtung der gegenüberliegenden Seite des Durchlasses und des stromabwärtigen Endes des Kanals (1) erstreckt.

4. Trinksystem nach einem der vorhergehenden Ansprüche, mit einem luftdicht geschlossenen Behälter (6) mit einem Durchlaß für den Kanal (1) und einem Lüftungsdurchlaß (8).

5. Trinksystem nach Anspruch 4, bei dem in dem Lüftungsdurchlaß (8) ein Filter (13) angeordnet ist.

6. Trinksystem nach einem der vorhergehenden Ansprüche, mit einer Umfangsausnehmung (17) auf der Außenseite der Wand der Kammer (3) und nahe dem stromabwärtigen Ende des Kanals (1).

7. Trinksystem nach einem der vorhergehenden Ansprüche, mit einer nach außen vorstehenden aufragenden Rippe (18) an der Außenwand der Kammer (3) und nahe dem stromabwärtigen Ende des Kanals (1).

8. Trinksystem nach einem der vorhergehenden Ansprüche, sich der Kanal durch eine Leitung und ein mit der Leitung verbundenes Mundstück erstreckt, wobei das Mundstück die Kammer (3) aufweist, in der das Rückschlagventil (2) angeordnet ist.

9. Mundstück zur Verwendung in einem Trinksystem nach Anspruch 8, mit einem Durchlaß (3, 4), der sich von einer Verbindungseinrichtung zum Verbinden einer Leitung mit einem stromabwärtigen Ende aus erstreckt, und einem Rückschlagventil (2), das ermöglicht, die Flüssigkeit ausschließlich in einer bestimmten Fließrichtung fließen zu lassen, wobei das Rückschlagventil (2) eine flexible Membran aufweist, und die Kammer (3) direkt dem stromabwärtigen Ende des Kanals (1) benachbart derart angeordnet ist, daß die Auslaßöffnung das stromabwärtige Ende des Kanals (1) bildet, wobei ein äußerer Bereich des Kanals (1) unmittelbar nahe dem stromabwärtigen Ende durch die die Kammer (3) begrenzenden Wände gebildet ist.

## Revendications

1. Système pour boissons comprenant un canal (1) de circulation d'un liquide provenant d'un récipient (6) vers la bouche d'un utilisateur et un clapet de retenue (2) placé dans le canal (1) et destiné a permettre le passage du liquide uniquement dans un sens particulier de circulation, le clapet de retenue (2) étant placé dans une chambre proche d'une extrémité aval du canal (1) considéré dans le sens de circulation, la chambre ayant une ouverture d'entrée et une ouverture de sortie et étant délimitée par des parois, caractérisé en ce que le clapet de retenue (2) comporte une membrane flexible, et la chambre (3) est placée afin qu'elle soit directement adjacente à l'extrémité aval du canal (1), si bien que l'ouverture de sortie forme une extrémité aval du canal (1), une partie externe du canal (1) adjacente directement à son extrémité aval étant formée par les parois délimitant la chambre (3).

2. Système pour boissons selon la revendication 1, comprenant un épaulement ayant un siège (5) contre lequel le clapet de retenue (2) est en butée à l'état de fermeture.

3. Système pour boissons selon la revendication 2, dans lequel la membrane (2) est raccordée d'un premier côté à la paroi de la chambre (3) dans laquelle elle est placée et, en position de fermeture, s étend depuis ce côté obliquement vers le côté opposé du passage et d'extrémité aval du canal (1).

4. Système pour boissons selon l'une quelconque des revendications précédentes, comprenant un récipient fermé hermétiquement (6) ayant un passage pour le canal (1) et un passage d'aération (8).

5. Système pour boissons selon la revendication 4, dans lequel un filtre (13) est placé dans le passage d'aération (8).

6. Système pour boissons selon l'une quelconque des revendications précédentes, comprenant une cavité circonférentielle (17) placée à l'extérieur de la paroi de la chambre (3) et près de l'extrémité aval du canal (1).

7. Système pour boissons selon l'une quelconque des revendications précédentes, comprenant une arête perpendiculaire (18) dépassant vers l'extérieur de la paroi de la chambre (3) et près de l'extrémité aval du canal (1).

8. Système pour boissons selon l'une quelconque des revendications précédentes, dans lequel le canal s'étend dans un conduit et un embout raccordé au conduit, l'embout contenant la chambre (3) dans laquelle est placé le clapet de retenue (2).

9. Embout destiné à être utilisé dans un système pour boissons selon la revendication 8, comprenant un passage (3, 4) qui s'étend depuis une connexion destinée à raccorder un conduit à une extrémité aval et un clapet de retenue (2) destiné à permettre le passage du liquide exclusivement dans un sens particulier de circulation, le clapet de retenue (2) comprenant une membrane flexible, et la chambre (3) étant placée afin qu'elle soit directement adjacente à l'extrémité aval du canal (1), si bien que l'ouverture de sortie forme l'extrémité aval du canal (1), une partie extérieure du canal (1) qui est directement adjacente à son extrémité aval étant formée par les parois qui délimitent la chambre (3).
